# EUROPEAN PATENT APPLICATION

(11) **EP 4 306 536 A1**
(43) Date of publication of application: **17.01.2024**
(21) Application number: 22796174.5
(22) Date of filing: 28.04.2022
(51) Int. Cl.: C07K 14/245, C12N 15/70, C12P 13/22

(54) **MUTANT SPOT PROTEIN AND METHOD FOR PRODUCING L-AMINO ACIDS USING SAME**

(30) Priority: 28.04.2021 KR 20210055169
(71) Applicant: CJ Cheiljedang Corporation, Seoul 04560 (KR)
(72) Inventor: KIM, Heejung, Seoul 04560 (KR); JUNG, Moo Young, Seoul 04560 (KR); KIM, Hye Mi, Seoul 04560 (KR); KIM, Hyun Ah, Seoul 04560 (KR)
(74) Representative: Icosa
(86) International application number: PCT/KR2022/006097
(87) International publication number: WO 2022/231342

(57) **Abstract**

Provided are a variant SpoT protein and a method of producing an L-amino acid using the same.

## Description

### BACKGROUND OF THE INVENTION

### 1. Field of the Invention

The present disclosure relates to a variant SpoT protein and a method of producing an L-amino acid using the same.

### 2. Description of the Related Art

For microorganisms used for the production of L-amino acids, originally, selected strains exhibiting analog resistance through chemical or physical mutation were mainly used, but use of recombinant strains using genetic engineering techniques has predominated due to the rapid development of genetic recombination technology and the establishment of molecular-level regulatory mechanisms in the 1990s.

Stringent response is a bacterial response to various stress situations, which was first discovered as a phenomenon in which protein synthesis is suppressed by amino acid starvation. Currently, it is known that stringent response is to adapt to various stress environments through changes in various physiological activities, such as cell division arrest, DNA replication inhibition, *etc.* (F.C. Neidhart, ed. (Washington, D.C.: ASM Press), (1996) pp. 1458-1496, J. Mol. Microbiol. Biotechnol. (2002) 4:331-340.). The key reactions of stringent response are initiated by guanosine pentphosphate (pppGpp) and guanosine tetraphosphate (ppGpp). Since there is no great functional difference between these two molecules, they are generally expressed as (p)ppGpp.

In *Escherichia coli,* pppGpp is produced by the pppGpp synthetase named RelA. The RelA protein monitors the translation of proteins on the ribosome. The intracellular protein deficiency increases the concentration of amino acid-uncharged tRNA, which binds to ribosomes, leading to activation of RelA. RelA produces pppGpp by reacting guanosine triphosphate (GTP) with adenosine triphosphate (ATP). When the intracellular amino acid concentration increases and balance is restored, (p)ppGpp is hydrolyzed by SpoT protein. SpoT protein has not only (p)ppGpp degradation activity but also synthesis activity (Nat Rev Microbiol. (2012) 10(3):203-12). However, the relationship between SpoT protein and amino acid production is still unknown.

The present disclosure provides a variant SpoT protein and a method of producing an L-amino acid using the same.

### SUMMARY OF THE INVENTION

An object of the present disclosure is to provide a variant SpoT protein including a substitution of an amino acid corresponding to position 262 in a sequence of SEQ ID NO: 1 with another amino acid.

Another object of the present disclosure is to provide a polynucleotide encoding the variant SpoT protein; and a vector including the same.

Still another object of the present disclosure is to provide a microorganism of the genus *Escherichia* including one or more of the variant SpoT protein; the polynucleotide encoding the variant SpoT protein; and the vector including the polynucleotide.

Still another object of the present disclosure is to provide a method of producing an L-amino acid, the method including culturing the microorganism in a medium.

Still another object of the present disclosure is to provide a method of increasing L-amino acid producing ability of a microorganism, the method including the step of modifying the microorganism to express the variant SpoT protein.

### DETAILED DESCRIPTION OF THE PREFERRED EMBODIMENTS

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a variant SpoT protein including a substitution of an amino acid corresponding to position 262 in a sequence of SEQ ID NO: 1 with another amino acid.

The variant SpoT protein refers to a variant protein including a substitution of the amino acid corresponding to position 262 from the N-terminus in the amino acid sequence of SEQ ID NO: 1 with another amino acid, in a polypeptide having the activity of SpoT protein or in SpoT protein.

In one embodiment, another amino acid may be an amino acid selected from glycine, valine, leucine, isoleucine, methionine, phenylalanine, tryptophan, proline, serine, threonine, cysteine, tyrosine, asparagine, glutamine, aspartic acid, glutamic acid, lysine, arginine, and histidine.

With regard to the variant protein according to any one of the above-described embodiments, another amino acid may be selected from polar amino acids.

With regard to the variant protein according to any one of the above-described embodiments, another amino acid may be selected from serine, threonine, cysteine, tyrosine, asparagine, and glutamine.

With regard to the variant protein according to any one of the above-described embodiments, the variant SpoT protein may have or include any one sequence of amino acid sequences represented by SEQ ID NOS: 19 to 24, or may essentially consist of the amino acid sequence.

With regard to the variant protein according to any one of the above-described embodiments, the variant SpoT protein may include substitution of the amino acid corresponding to positions 262, based on the amino acid sequence of SEQ ID NO: 1, with another amino acid, and may include an amino acid sequence having at least 70%, 75%, 80%, 85%, 90%, 95%, 96%, 97%, 98%, 99% or more, or less than 100% homology or identity to the amino acid sequence represented by SEQ ID NO: 1.

It is also apparent that a variant protein having an amino acid sequence having deletion, modification, substitution, conservative substitution, or addition of some sequence also falls within the scope of the present disclosure as long as the amino acid sequence has such a homology or identity and exhibits efficacy corresponding to that of the variant protein of the present disclosure.

Examples thereof include those having sequence addition or deletion that does not alter the function of the variant protein of the present disclosure at the N-terminus, C-terminus of the amino acid sequence, and/or inside the amino acid sequence, naturally occurring mutation, silent mutation, or conservative substitution.

As used herein, the term "conservative substitution" means substitution of one amino acid with another amino acid having similar structural and/or chemical properties. Such an amino acid substitution may generally occur based on similarity in the polarity, charge, solubility, hydrophobicity, hydrophilicity, and/or amphipathic nature of the residues. Usually, conservative substitution may hardly affect or not affect the activity of proteins or polypeptides.

The variant protein of the present disclosure may also be described as a "protein variant".

As used herein, the term "variant" refers to a polypeptide which has an amino acid sequence different from that of the variant before being varied by conservative substitution and/or modification of one or more amino acids but maintains the functions or properties. Such a variant may be generally identified by modifying one or more amino acids of the amino acid sequence of the polypeptide and evaluating the properties of the modified polypeptide. In other words, ability of the variant may be increased, unchanged, or decreased, as compared to that of the polypeptide before being varied. Further, some variants may include variants in which one or more portions such as an N-terminal leader sequence or a transmembrane domain have been removed. Other variants may include variants in which a portion of the N- and/or C-terminus has been removed from the mature protein. The term "variant" may be used interchangeably with terms such as modification, modified polypeptide, modified protein, mutant, mutein, and divergent, and is not limited thereto as long as it is a term used with the meaning of variation. With respect to the objects of the present disclosure, the variant may be a polypeptide including an amino acid sequence, in which the amino acid corresponding to position 262 in the amino acid sequence of SEQ ID NO: 1 is substituted with another amino acid.

Further, the variant may include deletions or additions of amino acids that have minimal effect on the properties and secondary structure of the polypeptide. For example, a signal (or leader) sequence that is co-translationally or post-translationally involved in the protein translocation may be conjugated to the N-terminus of the variant. The variant may be conjugated with other sequences or linkers so as to be identified, purified, or synthesized.

As used herein, the term "homology" or "identity" means the degree of similarity between two given amino acid sequences or base sequences, and may be expressed as a percentage. The terms "homology" and "identity" may often be used interchangeably.

The sequence homology or identity of a conserved polynucleotide or polypeptide is determined by standard alignment algorithms, and the default gap penalty established by a program used may be used together. Substantially, homologous or identical sequences are generally capable of being hybridized with the entirety or part of the sequence under moderately or highly stringent conditions. It is apparent that hybridization also includes hybridization with a polynucleotide including a general codon or a codon in consideration of codon degeneracy.

Whether or not any two polynucleotide or polypeptide sequences have homology, similarity, or identity may be determined using known computer algorithms such as the "FASTA" program, for example, using default parameters as in Pearson et al. (1988) Proc. Natl. Acad. Sci. USA 85:2444. Alternatively, the homology, similarity, or identity may be determined using Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (including GCG program package (Devereux, J., et al., Nucleic Acids Research 12:387 (1984)), BLASTP, BLASTN, FASTA (Atschul, S. F., et al., J MOLEC BIOL 215:403 (1990); Guide to Huge Computers, Martin J. Bishop, ed., Academic Press, San Diego,1994, and CARILLO et al. (1988) SIAM J Applied Math 48:1073). For example, BLAST of the National Center for Biotechnology Information or ClustalW may be used to determine the homology, similarity, or identity.

The homology, similarity, or identity of polynucleotides or polypeptides may be determined by comparing sequence information using, for example, a GAP computer program such as Needleman et al. (1970), J Mol Biol. 48:443 as announced in, for example, Smith and Waterman, Adv. Appl. Math (1981) 2:482. In summary, the GAP program may be defined as the value acquired by dividing the number of similarly aligned symbols (i.e., nucleotides or amino acids) by the total number of symbols in the shorter of two sequences. The default parameters for the GAP program may include (1) a binary comparison matrix (including values of 1 for identity and 0 for non-identity) and a weighted comparison matrix of Gribskov et al. (1986) Nucl. Acids Res. 14:6745 (or EDNAFULL (EMBOSS version of NCBI NUC4.4) substitution matrix) as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

For one example of the present disclosure, the variant SpoT protein of the present disclosure may have the activity of SpoT protein. Further, the variant SpoT protein of the present disclosure may have activity to increase the L-amino acid producing ability, as compared to a wild-type polypeptide having the activity of SpoT protein.

As used herein, the term "SpoT protein" is a polypeptide having (p)ppGpp degradation and/or synthesis activity. Specifically, the SpoT protein of the present disclosure may be used interchangeably with bifunctional (p)ppGpp synthase or bifunctional (p)ppGpp hydrolase. In the present disclosure, the sequence of the SpoT protein may be obtained from GenBank of NCBI, which is a known database. Specifically, the SpoT protein may be a polypeptide encoded by *spot* gene, but is not limited thereto.

In one embodiment, the SpoT protein of the present disclosure may be derived from the genus *Escherichia* (*Escherichia* sp.). In one embodiment, the SpoT protein of the present disclosure may be an amino acid sequence of SEQ ID NO: 1. In one embodiment, the SpoT protein of the present disclosure may include an amino acid sequence having 70% or more, 75%, 80%, 85%, 90%, 95%, 96%, 97%, 98%, or 99% or more homology or identity to the amino acid sequence of SEQ ID NO: 1, but is not limited thereto.

As used herein, the term "corresponding to" refers to amino acid residues at positions listed in the polypeptide or amino acid residues that are similar, identical, or homologous to those listed in the polypeptide. Identifying the amino acid at the corresponding position may be determining a specific amino acid in a sequence that refers to a specific sequence. As used herein, the "corresponding region" generally refers to a similar or corresponding position in a related protein or a reference protein.

For example, an arbitrary amino acid sequence is aligned with SEQ ID NO: 1, and based on this, each amino acid residue of the amino acid sequence may be numbered with reference to the numerical position of the amino acid residue corresponding to the amino acid residue of SEQ ID NO: 1. For example, a sequence alignment algorithm as described in the present disclosure may determine the position of an amino acid, or a position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48:443-453), the Needleman program of the EMBOSS package (EMBOSS: The European Molecular Biology Open Software Suite, Rice et al., 2000), Trends Genet. 16:276-277), *etc.* may be used, but are not limited thereto, and a sequence alignment program, a pairwise sequence comparison algorithm, *etc.,* which are known in the art, may be appropriately used.

Another aspect of the present disclosure provides a polynucleotide encoding the variant SpoT protein of the present disclosure.

As used herein, the term "polynucleotide" is a DNA or RNA strand having a certain length or more as a polymer of nucleotides in which nucleotide monomers are connected in a long chain by covalent bonds, and more specifically, means a polynucleotide fragment encoding the variant SpoT protein.

The polynucleotide encoding the variant SpoT protein of the present disclosure may include a nucleotide sequence encoding the amino acid sequence including the substitution of the amino acid corresponding to position 262 in the sequence of SEQ ID NO: 1 with another amino acid. In one embodiment, the polynucleotide of the present disclosure may include, consist of, or essentially consist of nucleotide sequences encoding amino acid sequences of SEQ ID NO: 19 to 24.

In the polynucleotide of the present disclosure, various modifications may be made in the coding region as long as the amino acid sequence of the variant protein of the present disclosure is not changed in consideration of codon degeneracy or codons preferred in organisms that are intended to express the variant protein of the present disclosure. Specifically, the polynucleotide of the present disclosure may have or include a base sequence having 70% or more, 75% or more, 80% or more, 85% or more, 90% or more, 95% or more, 96% or more, 97% or more, 98% or more, and less than 100% homology or identity to the sequence of SEQ ID NO: 2, or may consist of or essentially consist of a base sequence having 70% or more, 75% or more, 80% or more, 85% or more, 90% or more, 95% or more, 96% or more, 97% or more, 98% or more, and less than 100% homology or identity to the sequence of SEQ ID NO: 2, but is not limited thereto. Here, in the sequence having homology or identity, the codon encoding the amino acid corresponding to position 262 of SEQ ID NO: 1 may be one of the codons encoding amino acids excluding alanine, specifically, one of the codons encoding an amino acid selected from glycine, valine, leucine, isoleucine, methionine, phenylalanine, tryptophan, proline, serine, threonine, cysteine, tyrosine, asparagine, glutamine, aspartic acid, glutamic acid, lysine, arginine, and histidine.

Further, the polynucleotide of the present disclosure may include a probe that may be prepared from a known gene sequence, for example, a sequence without limitation as long as it is a sequence that is able to hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see Sambrook *et al., supra,* 9.50-9.51, 11.7-11.8). Examples thereof include a condition in which polynucleotides having higher homology or identity, i.e., polynucleotides having 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed once, specifically, twice to three times at a salt concentration and temperature equivalent to 60°C, 1X SSC, 0.1% SDS, specifically 60°C, 0.1X SSC, 0.1% SDS, more specifically 68°C, 0.1X SSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also include substantially similar nucleic acid sequences as well as isolated nucleic acid fragments that are complementary to the entire sequence.

Specifically, a polynucleotide having homology or identity to the polynucleotide of the present disclosure may be detected using a hybridization condition including a hybridization step at a Tₘ value of 55°C and the above-described conditions. Further, the Tₘ value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook *et al., supra*).

Still another aspect of the present disclosure provides a vector including the polynucleotide of the present disclosure. The vector may be an expression vector for expressing the polynucleotide in a host cell, but is not limited thereto.

As used herein, the term "vector" may include a DNA construct including a polynucleotide sequence encoding a polypeptide of interest which is operably linked to a suitable expression regulatory region (or expression control sequence) so that the polypeptide of interest may be expressed in a suitable host. The expression regulatory region may include a promoter capable of initiating transcription, any operator sequence for controlling the transcription, a sequence encoding a suitable mRNA ribosome binding site, and a sequence controlling termination of transcription and translation. The vector may be transformed into a suitable host cell and then replicated or function independently of the host genome, or may be integrated into the genome itself.

The vector used in the present disclosure is not particularly limited, but any vector known in the art may be used. Examples of commonly used vectors include natural or recombinant plasmids, cosmids, viruses, and bacteriophages. For example, pWE15, M13, MBL3, MBL4, IXII, ASHII, APII, t10, t11, Charon4A, Charon21A, *etc.* may be used as a phage vector or a cosmid vector, and pDZ system, pBR system, pUC system, pBluescript II system, pGEM system, pTZ system, pCL system, pET system, *etc.* may be used as a plasmid vector. Specifically, pDZ, pDC, pDCM2, pACYC177, pACYC184, pCL, pECCG117, pUC19, pBR322, pMW118, pCC1 BAC vector, *etc.* may be used.

For example, a polynucleotide encoding a polypeptide of interest may be inserted into a chromosome through a vector for intracellular chromosome insertion. Insertion of the polynucleotide into the chromosome may be performed by any method known in the art, for example, homologous recombination, but is not limited thereto. The vector may further include a selection marker for the confirmation of chromosome insertion. The selection marker is for selecting the cells transformed with vectors, i.e., for confirming the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector including a polynucleotide encoding a target polypeptide is introduced into a host cell or a microorganism so that the polypeptide encoded by the polynucleotide may be expressed in the host cell. The transformed polynucleotide may be located regardless of the position, either by being inserted into the chromosome of the host cell or located outside the chromosome as long as it may be expressed in the host cell. Further, the polynucleotide includes DNA and/or RNA encoding a polypeptide of interest. The polynucleotide may be introduced in any form as long as it may be introduced into a host cell and expressed. For example, the polynucleotide may be introduced into a host cell in the form of an expression cassette, which is a gene construct containing all elements required for self-expression. The expression cassette may usually include a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, the term "operably linked" means that the polynucleotide sequence is functionally linked to a promoter sequence that initiates and mediates transcription of the polynucleotide encoding the variant of interest of the present disclosure.

Still another aspect of the present disclosure provides a microorganism of the genus *Escherichia* (*Escherichia* sp.) including one or more of the variant SpoT protein of the present disclosure; the polynucleotide encoding the variant SpoT protein; and the vector including the polynucleotide.

The microorganism of the present disclosure may include the variant SpoT protein of the present disclosure, the polynucleotide encoding the variant protein, or the vector including the polynucleotide of the present disclosure.

In one embodiment, the microorganism of the present disclosure may express the variant SpoT protein of the present disclosure by including the variant SpoT protein of the present disclosure, the polynucleotide encoding the variant protein, or the vector including the polynucleotide of the present disclosure.

As used herein, the term "strain (or microorganism)" includes all of wild-type microorganisms or naturally or artificially genetically modified microorganisms, and it may be a microorganism in which a specific mechanism is weakened or strengthened due to insertion of a foreign gene or an activity enhancement or inactivation of an endogenous gene, and it may be a microorganism including genetic modification for production of a polypeptide, protein, or product of interest.

The strain of the present disclosure may be a strain including any one or more of the variant protein of the present disclosure, the polynucleotide of the present disclosure, and the vector including the polynucleotide of the present disclosure; a strain modified to express the variant protein of the present disclosure or the polynucleotide of the present disclosure; a strain (*e.g.,* a recombinant strain) expressing the variant protein of the present disclosure or the polynucleotide of the present disclosure; or a strain (*e.g.,* a recombinant strain) having the activity of the variant protein of the present disclosure, but is not limited thereto.

The strain of the present disclosure may be a strain having L-amino acid producing ability.

In one embodiment, the L-amino acid may be L-tryptophan.

The strain of the present disclosure may be a microorganism naturally having the SpoT protein or L-amino acid producing ability, or a microorganism in which the variant SpoT protein of the present disclosure or the polynucleotide encoding the same (or a vector including the polynucleotide) is introduced into and/or L-amino acid producing ability is conferred on a parent strain that does not have the SpoT protein or L-amino acid producing ability, but is not limited thereto.

For example, the strain of the present disclosure is a cell or microorganism that is transformed with the polynucleotide of the present disclosure or the vector including the polynucleotide encoding the variant protein of the present disclosure to express the variant protein of the present disclosure. The strain of the present disclosure may include any microorganism that is able to produce L-amino acids by including the variant of the present disclosure. For example, the strain of the present disclosure may be a recombinant strain in which the polynucleotide encoding the variant of the present disclosure is introduced into a natural wild-type microorganism or a microorganism producing an L-amino acid to express the SpoT variant and to have the increased L-amino acid producing ability. The recombinant strain having the increased L-amino acid producing ability may be a microorganism having the increased L-amino acid producing ability, as compared to a natural wild-type microorganism or a SpoT-unmodified microorganism (*i.e.,* a microorganism expressing the wild-type SpoT protein), but is not limited thereto. For example, the SpoT-unmodified microorganism, which is a target strain for comparing the increase in the L-amino acid producing ability, may be CA04-4303 or KCCM11166P strain, but is not limited thereto.

For example, the recombinant strain having the increased L-amino acid producing ability may have an increased L-amino acid producing ability of about 5% or more, 6% or more, or 8% or more, as compared to the L-amino acid producing ability of the parent strain before being varied or an unmodified microorganism, but the increased amount is not limited thereto as long as the producing ability has an increased amount of a + value, as compared to the producing ability of the parent strain before being varied or an unmodified microorganism. The term "about" refers to a range which includes all of ±0.5, ±0.4, ±0.3, ±0.2, ±0.1, *etc.,* and includes all of the values that are equivalent or similar to those following the term "about", but the range is not limited thereto.

As used herein, the term "unmodified microorganism" does not exclude strains including mutation that may occur naturally in microorganisms, and may be a wild-type strain or a natural strain itself or may be a strain before the trait is changed by genetic variation due to natural or artificial factors. For example, the unmodified microorganism may be a strain into which the variant SpoT protein described in the present specification is not introduced or has not yet been introduced. The term "unmodified microorganism" may be used interchangeably with "strain before being modified", "microorganism before being modified", "unvaried strain", "unmodified strain", "unvaried microorganism", or "reference microorganism".

For another example of the present disclosure, the microorganism of the present disclosure may be a microorganism of the genus *Escherichia.* Specifically, the microorganism may be *Escherichia coli,* but is not limited thereto.

With regard to the microorganism of the present disclosure, the variant SpoT protein, polynucleotide, L-amino acid, *etc.* are as described in other aspects.

Still another aspect of the present disclosure provides a method of producing an L-amino acid, the method including the step of culturing the microorganism of the genus *Escherichia* of the present disclosure in a medium.

The method of producing an L-amino acid of the present disclosure may include the step of culturing, in a medium, the strain of the genus *Escherichia* including the variant SpoT protein of the present disclosure or the polynucleotide of the present disclosure or the vector of the present disclosure.

In one embodiment, the L-amino acid may be L-tryptophan.

As used herein, the term "culturing" means growing the strain of the genus *Escherichia* of the present disclosure under appropriately controlled environmental conditions. The culturing process of the present disclosure may be performed according to suitable medium and culture conditions known in the art. Such a culture process may be easily adjusted and used by those skilled in the art according to the selected strain. Specifically, the culturing may be a batch type, continuous type, and/or fed-batch type, but is not limited thereto.

As used herein, the term "medium" means a mixed substance containing nutrients required to culture the strain of the genus *Escherichia* of the present disclosure as a main component, and the medium supplies nutrients, growth factors, *etc.,* including water, which are indispensable for survival and development. Specifically, as the medium and other culture conditions used for culturing the strain of the genus *Escherichia* of the present disclosure, any one may be used without particular limitation as long as it is a medium used for common culture of microorganisms. The strain of the genus *Escherichia* of the present disclosure may be cultured in a common medium containing proper carbon sources, nitrogen sources, phosphorus sources, inorganic compounds, amino acids and/or vitamins, *etc.,* while controlling the temperature, pH, *etc.* under aerobic conditions.

In the present disclosure, the carbon sources include carbohydrates such as glucose, saccharose, lactose, fructose, sucrose, maltose, *etc*.; sugar alcohols such as mannitol, sorbitol, *etc.,* organic acids such as pyruvic acid, lactic acid, citric acid, *etc*.; amino acids such as glutamic acid, methionine, lysine, *etc.* Natural organic nutrients such as starch hydrolysate, molasses, blackstrap molasses, rice bran, cassava, sugarcane residue, and corn steep liquor may be used. Specifically, carbohydrates such as glucose and sterilized pretreated molasses (i.e., molasses converted to reducing sugar) may be used, and appropriate amounts of other carbon sources may be used in various manners without limitation. These carbon sources may be used singly or in combination of two or more thereof, but are not limited thereto.

As the nitrogen sources, inorganic nitrogen sources such as ammonia, ammonium sulfate, ammonium chloride, ammonium acetate, ammonium phosphate, ammonium carbonate, ammonium nitrate, *etc*.; and organic nitrogen sources such as amino acids such as glutamic acid, methionine, glutamine, *etc.,* peptone, NZ-amine, meat extract, yeast extract, malt extract, corn steep liquor, casein hydrolysate, fish or decomposition products thereof, and skim soybean cake or decomposition products thereof may be used. These nitrogen sources may be used singly or in combination of two or more thereof, but are not limited thereto.

The phosphorus sources may include monopotassium phosphate, dipotassium phosphate, or sodium-containing salts corresponding thereto. As the inorganic compounds, sodium chloride, calcium chloride, iron chloride, magnesium sulfate, iron sulfate, manganese sulfate, calcium carbonate, *etc.* may be used. In addition, amino acids, vitamins and/or suitable precursors, *etc.* may be included. These components or precursors may be added to the medium batchwise or continuously, but is not limited thereto.

Further, during the culturing of the strain of the genus *Escherichia* of the present disclosure, the pH of the medium may be adjusted by adding compounds such as ammonium hydroxide, potassium hydroxide, ammonia, phosphoric acid, sulfuric acid, *etc.* to the medium in a proper manner. Further, during the culturing, foaming may be suppressed by using an antifoaming agent such as fatty acid polyglycol ester. Further, oxygen or oxygen-containing gas may be injected into the medium in order to maintain the aerobic state of the medium, or gas may not be injected, or nitrogen, hydrogen, or carbon dioxide gas may be injected in order to maintain the anaerobic and microaerobic states, but is not limited thereto.

In the culturing of the present disclosure, the culture temperature may be maintained at 20°C to 45°C, specifically at 25°C to 40°C, and the strain may be cultured for about 10 to 160 hours, but these are not limited thereto.

The L-amino acid produced through the culturing of the present disclosure may be secreted into the medium or may remain in the cells.

The method of producing an L-amino acid of the present disclosure may further include the steps of preparing the strain of the genus *Escherichia* of the present disclosure, preparing a medium for culturing the strain, or a combination thereof (in any order), for example, prior to the culturing step.

The method of producing an L-amino acid of the present disclosure may further include the step of recovering the L-amino acid from the medium according to the culturing (the medium subjected to the culturing) or from the strain of the genus *Escherichia* of the present disclosure. The recovering step may be further included after the culturing step.

The recovering may be to collect an L-amino acid of interest by way of a suitable method known in the art according to the method of culturing the microorganism of the present disclosure, for example, a batch, continuous, or fed-batch culture method. For example, centrifugation, filtration, treatment with a crystallized protein precipitant (salting out), extraction, sonication, ultrafiltration, dialysis, various forms of chromatography such as molecular sieve chromatography (gel filtration), adsorption chromatography, ion-exchange chromatography, and affinity chromatography, *etc.,* HPLC, or a combination thereof may be used. The L-amino acid of interest may be recovered from the medium or microorganism by way of a suitable method known in the art.

The method of producing an L-amino acid of the present disclosure may further include a purification step. The purification may be performed by way of a suitable method known in the art. In an example, when the method of producing an L-amino acid of the present disclosure includes both the recovery step and the purification step, the recovery step and the purification step may be performed discontinuously (or continuously) regardless of the order, or may be performed simultaneously or by being combined into one step, but is not limited thereto.

In the method of the present disclosure, the variant SpoT protein, polynucleotide, vector, strain, *etc.* are as described in other aspects.

Still another aspect of the present disclosure provides a composition for producing an L-amino acid, the composition including the variant SpoT protein of the present disclosure.

In one embodiment, the composition may include the variant SpoT protein of the present disclosure, the polynucleotide encoding the variant SpoT protein, the vector including the polynucleotide, or the strain of the genus *Escherichia* including the polynucleotide of the present disclosure; a culture medium, in which the strain is cultured; or a combination of two or more thereof.

The composition of the present disclosure may further include arbitrary suitable excipients which are commonly used in compositions for producing amino acids. Such excipients may be, for example, a preservative, a wetting agent, a dispersing agent, a suspending agent, a buffering agent, a stabilizer, or an isotonic agent, *etc.,* but are not limited thereto.

In the composition of the present disclosure, the variant SpoT protein, polynucleotide, vector, strain, medium, L-amino acid, *etc.* are as described in other aspects.

Still another aspect of the present disclosure provides a method of increasing the L-amino acid producing ability of a microorganism, the method including the step of modifying the microorganism to express the variant SpoT protein of the present disclosure.

Still another aspect of the present disclosure provides use of the variant SpoT protein in the production of L-amino acids.

The variant SpoT protein, microorganism, L-amino acids, *etc.* are as described in other aspects.

Meanwhile, throughout this specification, unless the context requires otherwise, the expression "include" "including", "containing", *etc.* will be understood to imply the inclusion of a stated integer or group of integers but not the exclusion of any other integer or group of integers.

Hereinafter, the present disclosure will be described in more detail by way of exemplary embodiments. However, these exemplary embodiments are only for illustrating the present disclosure, and thus the scope of the present disclosure is not limited to these exemplary embodiments.

### Example 1. Construction of recombinant vector pSKH-spoT

To obtain about 1.63 kb of a DNA fragment containing a mutation site of gene *spoT* (SEQ ID NO: 2), chromosomal DNA (gDNA) of wild-type *Escherichia coli* W3110 was extracted using Qiagen's Genomic-tip system, and polymerase chain reaction (PCR) was performed using the gDNA as a template and using a PCR HL premix kit (BIONEER's product, same hereafter). To amplify a gene fragment region, in which alanine which is an amino acid at position 262 of the SpoT amino acid sequence (SEQ ID NO: 1) was substituted with another polar amino acid, such as serine (S), threonine (T), cysteine (C), tyrosine (Y), asparagine (N), glutamine (Q), PCR was performed by repeating 27 cycles consisting of denaturation at 95°C for 30 seconds, annealing at 56°C for 30 seconds, and elongation at 68°C for 1 minute using primer sets of Table 1.

As a result, 0.82 kb and 0.88 kb of gene fragments, respectively, were obtained for the substitution mutation.

**[Table 1]**

| Mutagenic primer sets for construction of variant plasmids with amino acid variation at position 262 of SpoT amino acid sequence for introduction into the genus *Escherichia* | | |
|---|---|---|
| | SEQ ID NO | Nucleotide sequence |
| A262S-1 | 5 | CAGGAATTCGATGTCGCCCTTGTATCTGTTTGAAAGCCT |
| | 7 | CATTGACGATCACGCGGAA**AGA**GTAGATGTCCATGA |
| A262S-2 | 8 | TCGATCATGGACATCTAC**TCT**TTCCGCGTGATCGTCA |
| | 6 | GCTAGGTCGACTAGCGTGATCCCCATGTTGCAGATTTT |
| A262T-1 | 5 | CAGGAATTCGATGTCGCCCTTGTATCTGTTTGAAAGCCT |
| | 9 | CATTGACGATCACGCGGAA**AGT**GTAGATGTCCATGA |
| A262T-2 | 10 | TCGATCATGGACATCTAC**ACT**TTCCGCGTGATCGTCA |
| | 6 | GCTAGGTCGACTAGCGTGATCCCCATGTTGCAGATTTT |
| A262C-1 | 5 | CAGGAATTCGATGTCGCCCTTGTATCTGTTTGAAAGCCT |
| | 11 | CATTGACGATCACGCGGAA**ACA**GTAGATGTCCATGA |
| A262C-2 | 12 | TCGATCATGGACATCTAC**TGT**TTCCGCGTGATCGTCA |
| | 6 | GCTAGGTCGACTAGCGTGATCCCCATGTTGCAGATTTT |
| A262Y-1 | 5 | CAGGAATTCGATGTCGCCCTTGTATCTGTTTGAAAGCCT |
| | 13 | CATTGACGATCACGCGGAA**ATA**GTAGATGTCCATGA |
| A262Y-2 | 14 | TCGATCATGGACATCTAC**TAT**TTCCGCGTGATCGTCA |
| | 6 | GCTAGGTCGACTAGCGTGATCCCCATGTTGCAGATTTT |
| A262N-1 | 5 | CAGGAATTCGATGTCGCCCTTGTATCTGTTTGAAAGCCT |
| | 15 | CATTGACGATCACGCGGAA**ATT**GTAGATGTCCATGA |
| A262N-2 | 16 | TCGATCATGGACATCTAC**AAT**TTCCGCGTGATCGTCA |
| | 6 | GCTAGGTCGACTAGCGTGATCCCCATGTTGCAGATTTT |
| A262Q-1 | 5 | CAGGAATTCGATGTCGCCCTTGTATCTGTTTGAAAGCCT |
| | 17 | CATTGACGATCACGCGGAA**TTG**GTAGATGTCCATGA |
| A262Q-2 | 18 | TCGATCATGGACATCTAC**CAA**TTCCGCGTGATCGTCA |
| | 6 | GCTAGGTCGACTAGCGTGATCCCCATGTTGCAGATTTT |

The amplified variant spoT gene fragment and a pSKH vector (US 8569066 B2) for chromosomal transformation, which was digested with EcoR V restriction enzyme, were cloned using Gibson assembly (DG Gibson et al., NATURE METHODS, Vol. 6 No. 5, MAY 2009, NEBuilder HiFi DNA Assembly Master Mix) method to obtain recombinant plasmids, which were named pSKH-spoT(A262S), pSKH-spoT(A262T), pSKH-spoT(A262C), pSKH-spoT(A262Y), pSKH-spoT(A262N), and pSKH-spoT(A262Q) according to the substituted mutations. Cloning was performed by mixing Gibson assembly reagent and each of the gene fragments in a calculated number of moles, followed by incubating at 50°C for 1 hour.

### Example 2. Development of mutant strain in which coding sequence of amino acid at position 262 of SpoT is substituted with coding sequence of polar amino acid

Each of pSKH-spoT(A262S), pSKH-spoT(A262T), pSKH-spoT(A262C), pSKH-spoT(A262Y), pSKH-spoT(A262N), and pSKH-spoT(A262Q) obtained in Example 1 was transformed into electro-competent cells of an L-tryptophan-producing strain KCCM11166P (US 8835154 B2) by electroporation, and then strains were obtained through a second crossover process, in which the coding sequence of alanine which is an amino acid at position 262 of SpoT on the chromosome was substituted with a coding sequence of a polar amino acid, serine, threonine, cysteine, tyrosine, asparagine, or glutamine (SEQ ID NOS: 19-24). The corresponding genetic manipulation was confirmed through PCR and genome sequencing using primers of SEQ ID NO: 3 and SEQ ID NO: 4, which are able to amplify the external regions of the homologous recombinant upstream and downstream regions where the corresponding gene was inserted, respectively.
SEQ ID NO: 3 (spoT-confirm forward)
   CCAGGAACAGCAAGAGCA
SEQ ID NO: 4 (spoT-confirm reverse)
   ACGGATATTACGGCAGGA

The strains thus obtained were named *Escherichia coli* KCCM11166P::spoT(A262S), KCCM11166P::spoT(A262T), KCCM11166P::spoT(A262C), KCCM11166P::spoT(A262Y), KCCM11166P::spoT(A262N), KCCM11166P::spoT(A262Q), respectively.

In addition, whether the introduction of the SpoT 262 mutation showed the same effect in other L-tryptophan-producing strains of the genus *Escherichia* was examined in another L-tryptophan-producing strain, CA04-4303 (US 2020-0063219 A1). CA04-4303 is a strain prepared by deleting trpR, mtr, and tnaA and substituting an amino acid proline at position 21 of trpE with serine in the wild-type W3110 strain. Strains were prepared, in which the coding sequence of alanine which is an amino acid at position 262 of SpoT on the chromosome of CA04-4303 strain was substituted with a coding sequence of a polar amino acid, serine, threonine, cysteine, tyrosine, asparagine, or glutamine. The preparation procedure is the same as in the above introduction procedure of KCCM11166P.

The strains thus obtained were named CA04-4303::spoT(A262S), CA04-4303::spoT(A262T), CA04-4303::spoT(A262C), CA04-4303::spoT(A262Y), CA04-4303::spoT(A262N), CA04-4303::spoT(A262Q), respectively.

### Example 3. Comparison of L-tryptophan producing ability between mutant strains in which amino acid at position 262 of SpoT is substituted with polar amino acid

The strains prepared in Example 2 by substituting the amino acid at position 262 of SpoT with a polar amino acid, based on the L-tryptophan-producing strain KCCM11166P, were cultured in a tryptophan titration medium prepared according to a composition shown in Table 2 below, and L-tryptophan producing ability was examined.

**[Table 2]**

| Composition of tryptophan titration medium | |
|---|---|
| Composition | Concentration (per liter) |
| Glucose | 60 g |
| K₂HPO₄ | 1 g |
| (NH₄)₂SO₄ | 10 g |
| NaCl | 1 g |
| MgSO₄·7H₂O | 1 g |
| Sodium citrate | 5 g |
| Yeast extract | 2 g |
| Calcium carbonate | 40 g |
| Phenylalanine | 0.15 g |
| Tyrosine | 0.1 g |
| pH | 6.8 |

In detail, each one platinum loop of *Escherichia coli* KCCM11166P and *Escherichia coli* KCCM11166P::spoT(A262S), KCCM11166P::spoT(A262T), KCCM11166P::spoT(A262C), KCCM11166P::spoT(A262Y), KCCM11166P::spoT(A262N), and KCCM11166P::spoT(A262Q) cultured overnight on an LB solid medium in a 37°C incubator was inoculated into 25 mL of the titration medium of Table 2, and then cultured in an incubator at 37°C and 200 rpm for 48 hours, and the sugar consumption rates and L-tryptophan concentrations were compared.

As a result, as shown in Table 3 below, the sugar consumption of the control KCCM11166P strain which was observed at 22 hours of culture was 25.0 g/L, whereas the mutant strains in which the amino acid at position 262 of the amino acid sequence of SpoT protein was substituted with a polar amino acid showed the sugar consumption of 27 g/L to 31 g/L for the same amount of time. This is a value of 8.8% to 25.8% improved productivity, and in particular, it was confirmed that KCCM11166P::spoT(A262T) showed the greatest improvement in the productivity.

These results confirm that the amino acid at position 262 of SpoT protein is the main site for changing the activity of the corresponding protein, and substitution of the amino acid at position 262 with a polar amino acid may greatly improve productivity in the L-tryptophan fermentation.

**[Table 3]**

| Comparison of titers of tryptophan strains including SpoT variants | | | | |
|---|---|---|---|---|
| Strain | Consumed sugar (g/L)* | Tryptophan (g/L)* | Productivity* (g/L/h) | Tryptophan (g/L)** |
| KCCM11166P | 25.0 | 3.5 | 0.159 | 8.1 |
| KCCM11166P::spoT(A262S) | 29.5 | 4.2 | 0.191 | 8.3 |
| KCCM11166P::spoT(A262T) | 31.0 | 4.4 | 0.200 | 8.2 |
| KCCM11166P::spoT(A262C) | 27.0 | 3.8 | 0.173 | 8.1 |
| KCCM11166P::spoT(A262Y) | 29.2 | 4.1 | 0.186 | 8.3 |
| KCCM11166P::spoT(A262N) | 39.6 | 4.2 | 0.191 | 8.1 |
| KCCM11166P::spoT(A262Q) | 27.8 | 3.9 | 0.177 | 8.2 |

| | | | | |
|---|---|---|---|---|
| *22-hour measurements, ** 48-hour measurements | | | | |

The change in the L-tryptophan producing ability due to the substitution of the amino acid at position 262 of SpoT with a polar amino acid was confirmed again in the CA04-4303 strain obtained in Example 2. The culture method and medium composition for comparison of the L-tryptophan producing ability are the same as those of the KCCM11166P-based culture.

As seen from the results below, the mutations selected in the present disclosure greatly improved L-tryptophan productivity even in the substitution of the amino acid at position 262 of SpoT, based on the L-tryptophan-producing strain CA04-4303 strain.

As shown in Table 4 below, the sugar consumption of the control CA04-4303 strain which was observed at 22 hours of culture was 30.0 g/L, whereas the mutant strains in which the amino acid at position 262 of the amino acid sequence of SpoT protein was substituted with a polar amino acid showed the sugar consumption of 31.8 g/L to 33.0 g/L for the same amount of time. This is a value of 13.0% to 30.4% improved productivity, and in particular, it was confirmed that CA04-4303::spoT(A262Y) showed the greatest improvement in the productivity.

**[Table 4]**

| Growth of wild-type strain and evaluation result of tryptophan producing ability | | | | |
|---|---|---|---|---|
| Strain | Consumed sugar (g/L)* | Tryptophan (g/L)* | Productivity* (g/L/h) | Tryptophan (g/L)** |
| CA04-4303 | 30.0 | 0.50 | 0.023 | 1.3 |
| CA04-4303(spoT A262S) | 33.1 | 0.60 | 0.027 | 1.5 |
| CA04-4303(spoT A262T) | 33.0 | 0.64 | 0.029 | 1.4 |
| CA04-4303(spoT A262C) | 31.8 | 0.58 | 0.026 | 1.3 |
| CA04-4303(spoT A262Y) | 32.0 | 0.67 | 0.030 | 1.4 |
| CA04-4303(spoT A262N) | 32.9 | 0.62 | 0.028 | 1.5 |
| CA04-4303(spoT A262Q) | 32.5 | 0.59 | 0.027 | 1.4 |

| | | | | |
|---|---|---|---|---|
| *22-hour measurements, ** 48-hour measurements | | | | |

The above results indicate that the L-amino acid productivity is increased in the microorganisms of the genus *Escherichia,* which are introduced with the variant SpoT in which the amino acid at position 262 of the amino acid sequence of the present disclosure is substituted with a polar amino acid, resulting in an increase in the L-amino acid productivity, as compared to unmodified strains.

Based on the above description, it will be understood by those skilled in the art that the present disclosure may be implemented in a different specific form without changing the technical idea or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

### Effect of the Invention

L-Amino acids may be effectively produced using a variant protein of the present disclosure.

## Claims

1. A variant SpoT protein comprising a substitution of an amino acid corresponding to position 262 in a sequence of SEQ ID NO: 1 with another amino acid.

2. The variant SpoT protein of claim 1, wherein another amino acid is selected from polar amino acids.

3. The variant SpoT protein of claim 1, wherein another amino acid is selected from serine, threonine, cysteine, tyrosine, asparagine, and glutamine.

4. A polynucleotide encoding the variant SpoT protein of any one of claims 1 to 3.

5. A microorganism of the genus *Escherichia* comprising one or more of the variant SpoT protein of any one of claims 1 to 3 and a polynucleotide encoding the variant SpoT protein.

6. The microorganism of claim 5, wherein the microorganism of the genus *Escherichia* is *Escherichia coli.*

7. The microorganism of claim 5, wherein the microorganism of the genus *Escherichia* produces an L-amino acid.

8. The microorganism of claim 7, wherein the L-amino acid is L-tryptophan.

9. A method of producing an L-amino acid, the method comprising the step of culturing the microorganism of the genus *Escherichia* of claim 5 in a medium.

10. The method of claim 9, wherein the L-amino acid is L-tryptophan.

11. The method of claim 9, comprising the step of recovering the L-amino acid from the microorganism or medium.

12. A composition for producing an L-amino acid, the composition comprising the variant SpoT protein of any one of claims 1 to 3, a microorganism of the genus *Escherichia* expressing the variant SpoT protein, or a culture of the microorganism.

13. A method of increasing L-amino acid producing ability of a microorganism, the method comprising the step of modifying the microorganism to express the variant SpoT protein of any one of claims 1 to 3.
